# EUROPÄISCHE PATENTSCHRIFT

(11) **EP 1 694 215 B1**
(45) Veröffentlichungstag und Bekanntmachung des Hinweises auf die Patenterteilung: **13.01.2010**
(21) Anmeldenummer: 04798069.3
(22) Anmeldetag: 24.11.2004
(51) Int. Cl.: A61B 17/02, A61F 2/46

(54) **INSTRUMENTARIUM ZUM EINSETZEN EINER ZWISCHENWIRBEL-GELENKPROTHESE**
INSTRUMENT FOR THE INSERTION OF AN INTERVERTEBRAL ARTICULAR PROSTHESIS
INSTRUMENT D'INSERTION D'UNE PROTHESE ARTICULAIRE INTERVERTEBRALE

(30) Priorität: 10.12.2003 US 731432; 10.12.2003 US 731431
(43) Veröffentlichungstag der Anmeldung: 30.08.2006
(60) Teilanmeldung: 07006418.3
(73) Patentinhaber: Cervitech, Inc., San Diego CA 92121 (US)
(72) Erfinder: LINK, Helmut D., 22397 Hamburg (DE); KELLER, Arnold, 23863 Kayhude (DE); MCAFEE, Paul C., Baltimore, MD 21204 (US)
(74) Vertreter: Glawe, Delfs, Moll
(86) Internationale Anmeldenummer: PCT/EP2004/013346
(87) Internationale Veröffentlichungsnummer: WO 2005/055835

(56) Entgegenhaltungen:
- WO-A-01/62166
- WO-A-97/38635
- WO-A-03/075774
- WO-A-2004/089258
- US-A1- 2002 026 191
- US-A1- 2002 058 944
- US-A1- 2003 045 938

## Beschreibung

Beim Einsetzen einer Zwischenwirbel-Gelenkprothese zum Ersatz einer Bandscheibe arbeitet der Operateur in einem sehr unübersichtlichen Operationsfeld in unmittelbarer Nähe bedeutender Nerven- und Blutbahnen. Das gilt insbesondere für den Bereich der Halswirbelsäule, weil dort die Wirbelabmessungen sehr klein und die Abstände zu sensiblen Nachbarbereichen besonders gering sind. Man ist deshalb bestrebt, die Bewegungsfreiheit von Instrumenten, die ein besonderes Gefährdungspotential haben oder die mit besonderer Genauigkeit eingesetzt werden müssen, durch geeignete Instrumente auf den notwendigen Rahmen einzuschränken. Dennoch soll dabei die Sichtkontrolle weitestgehend möglich bleiben.

Es sind Instrumente zum Einsetzen von Zwischenwirbelprothesen in die Lendenwirbelsäule bekannt (DE-U-299 16 078, EP-A-0 333 990, FR-A-2737656), bei denen zunächst die Deckplatten einer Prothese in den Zwischenwirbelraum eingetrieben, gespreizt und anschließend der Prothesenkern zwischengeschoben wird. Auf eine Bearbeitung der Wirbeloberflächen kann dabei in der Regel verzichtet werden. Die Einsetzinstrumente sind verhältnismäßig groß, was aber im Bereich der Lendenwirbelsäule tolerabel ist. Im Bereich der Halswirbelsäule sind die Zwischenwirbelräume so eng, daß durch Bearbeiten der benachbarten Wirbelkörper Platz für die Aufnahme der Prothesen geschaffen werden muß. Dabei ist der Zugangsraum so eng und wegen der Nachbarschaft lebenswichtiger Organe so empfindlich, daß große Instrumente nicht eingesetzt werden können. Zur Vermeidung dieser Nachteile wurde das der Bildung des Oberbegriffs des Anspruchs 1 zugrundeliegende, bekannte Instrumentarium (WO 03/075774) entworfen. Es umfaßt eine Einrichtung zum gegenseitigen Fixieren der Wirbelkörper und zum Führen eines Werkzeugs sowie ein Justierinstrument zum Justieren der gegenseitigen Position der Wirbelkörper und der Fixier- und Führungseinrichtung. Das Justierinstrument besteht aus einer zwischen die Wirbelkörper zu schiebenden Zwischenwirbelplatte und einer damit verbundenen Justierstange. Auf dieser ist die Fixier- und Führungseinrichtung verschiebbar, die unterhalb und oberhalb der Justierstange Bohrungen aufweist, die zunächst als Führung für ein Bohrinstrument und anschließend für die Aufnahme von Haltemitteln, nämlich Knochenschrauben, dienen, durch die die Fixier- und Führungseinrichtung fest mit den Wirbelkörpern verbunden wird. Dadurch werden die Wirbelkörper in einer durch die Dicke der Zwischenwirbelplatte vorbestimmten gegenseitigen Stellung fixiert. Eine nachträgliche Korrektur ist kaum möglich. Diesen Nachteil haben auch weitere bekannte Instrumentarien (US 2002/0058944, US 2002/0026191, EP 1259175 B1, WO 01/62166), bei denen die Wirbelkörper in starrem Abstand zueinander fixiert werden. Außerdem erschweren sie die Sicht auf das Operationsfeld.

Diese Nachteile will die Erfindung vermeiden und erreicht dies durch die Mittel des Anspruchs 1. Neben der Änderbarkeit des Wirbelkörperabstands werden hohe Genauigkeit und weitestgehende Sichtkontrolle ermöglicht.

Bei der Benutzung des Instrumentariums wird zunächst die Zwischenwirbelplatte eingesetzt, nachdem die Bandscheibe und gegebenenfalls die ventralen Randzacken des oberen Wirbelkörpers entfernt wurden. Nach ihrer Positionierung gibt sie einen genauen Hinweis auf die Lage der Wirbelkörperoberflächen, zwischen denen die Prothese plaziert werden soll. Sie sorgt auch dafür, daß die Wirbelkörper den vorbestimmten Abstand voneinander haben. Sie wird zweckmäßigerweise so ausgewählt, daß sie etwa dieselbe Form und Abmessung wie der natürliche Zwischenwirbelraum hat bzw. daß ihre Ausdehnung nur wenig geringer ist als diejenige des Zwischenwirbelraums. Das erleichtert ihre Positionierung, weil sie selbsttätig aufgrund ihrer Form eine in bezug auf den Zwischenwirbelraum mittige und gleichgerichtete Stellung einnimmt. Sie kann auch mit Röntgenkontrollmarken zur genaueren Positionierung versehen sein. Sie wird in der vorgesehenen Stellung durch die Spannung gehalten, die von den natürlichen Bändern zwischen den Wirbelkörpern erzeugt wird. Diese Spannung hängt von der Dicke der Zwischenwirbelplatte ab. Eine hinreichende Spannung herrscht jedenfalls, wenn diese Dicke etwa so groß wie die Dicke der vorgesehenen Prothese gewählt wird. Damit ihre Position nachträglich noch korrigiert werden kann, ist ihre Oberfläche im wesentlichen glatt, d.h. ohne Erhebungen, die durch Einsinken in die Knochen- oder Knorpeloberfläche eine Relativbewegung parallel zur Oberflächenrichtung erschweren.

Die Führungseinrichtung wird von der Justierstange getragen, die vorne von der Zwischenwirbelplatte vorragt. Da die Position der Justierstange durch die Zwischenwirbelplatte vorbestimmt ist, erhält man auch einen Maßstab für die Position der Führungseinrichtung. Diese kann also mit Hilfe des Justierinstruments exakt, d.h. mit derselben Genauigkeit wie das Justierinstrument selbst, positioniert werden. Die gegenseitige Position der Wirbelkörper ist dann durch die Zwischenwirbelplatte ebenso gesichert wie die Position der Bearbeitungswerkzeuge durch die Führungseinrichtung. Die Bearbeitung der Wirbelkörper mit Hilfe der Führungseinrichtung findet statt, solange diese noch mit dem Justierinstrument verbunden und von diesem in der genauen Position gehalten wird.

Vorzugsweise ist die Führungseinrichtung mit einer Nabe vom freien Ende der Justierstange her auf diese aufschiebbar. Dies ermöglicht es dem Operateur, die zusammenwirkenden Justierflächen der Stange und der Führungseinrichtung in einem vorderen, übersichtlichen Teil des Operationsfeldes zusammenzustecken statt in der unübersichtlichen Tiefe. Die Führungseinrichtung wird dann entlang der Justierstange in die Tiefe des Operationsfeldes geführt.

Die Justierstange und die Führungseinrichtung haben zweckmäßigerweise undrehbar komplementär zueinander passend geformte Zusammenwirkungsflächen, damit sich die Führungseinrichtung nicht relativ zur Justierstange verdrehen kann. Die Führungseinrichtung weist zweckmäßigerweise eine Führung für zwei in der Medianebene parallel zur Justierstange oberhalb und unterhalb derselben angeordnete Führungsachsen auf. Beispielsweise kann je eine Bohrlehre unterhalb und oberhalb der die Justierstange umfassenden Nabe der Führungseinrichtung angeordnet sein.

Noch vorteilhafter ist eine Ausführung, bei welcher an der die Justierstange umfassenden Nabe nur eine Führung angeordnet ist und die Zusammenwirkungsflächen der Nabe und der Justierstange in zwei um 180° zueinander versetzten Stellungen zusammen passen. Dadurch wird das Instrument kleiner und die Übersicht über das Operationsfeld entsprechend verbessert. In einer ersten dieser beiden Stellungen wird die Führungseinrichtung dazu benutzt, einen ersten der beiden Wirbelkörper zu bearbeiten und gegebenenfalls ein Haltemittel (vorzugsweise einen Stift) darin zu verankern. Danach wird die Führungseinrichtung auf der Justierstange bis zu dessen runden Querschnittsteil zurückgezogen, um 180° gedreht und wieder vorgeschoben, um dieselbe Bearbeitung des anderen Wirbelkörpers zu ermöglichen.

Es kann dann ein Distraktionswerkzeug an den Haltemitteln angesetzt werden, das sie bei der Distraktion richtungsfest hält, d.h. die Haltemittel sind so mit dem Distraktionswerkzeug verbunden, daß die Haltemittel und mit ihnen die Wirbelkörper ihre gegenseitige Richtungseinstellung beibehalten. Vorzugsweise sind die Führungsachsen der Führungen sowie die dementsprechend eingebrachten Haltemittel bzw. Stifte parallel zueinander. Dann ordnet man entsprechend auch die an dem Distraktionsinstrument vorgesehenen Aufnahmen für die Haltemittel bzw. Stifte parallel zueinander an.

In manchen Fällen ist die natürliche Kyphose (Krümmung der Halswirbelsäule mit dorsal liegender Krümmungsachse) verringert. In diesen Fällen ist es zweckmäßig, die Wirbelkörper vor dem Einsetzen der Zwischenwirbelprothese nicht nur zu distrahieren, sondern auch die natürliche Kyphose wiederherzustellen. Dies gelingt erfindungsgemäß dadurch, daß die Zwischenwirbelplatte keilförmig ist, d.h. ihre Dicke verringert sich von ihrem ventralen Rand hin zu ihrem dorsalen Rand. Es können Zwischenwirbelplatten mit unterschiedlichem Keilwinkel in dem Instrumentarium bereitgehalten werden.

Die Erfindung ermöglicht ein Verfahren zum Einsetzen einer Zwischenwirbelprothese in den Zwischenwirbelraum zwischen zwei Wirbelkörpern, bei dem in einem ersten Schritt die Bandscheibe entfernt wird, in einem zweiten Schritt die Zwischenwirbelplatte eines Justierinstruments in dem Zwischenwirbelraum positioniert und darin festgeklemmt wird, in einem dritten Schritt die Nabe einer Führungseinrichtung auf eine von der zwischenwirbelplatte vorragende Justierstange derart aufgeschoben wird, daß sie zwei Führungsachsen in der Medianebene oberhalb und unterhalb der Justierstange definiert, in einem vierten Schritt die Stifte in Richtung der Führungsachsen in die Wirbel eingebracht werden, in einem fünften Schritt eine Distraktionszange mit den Stiften derart verbunden wird, daß sie parallel zueinander gehalten sind, und in weiteren Schritten der gegenseitige Abstand der Wirbelkörper eingestellt, die Führungseinrichtung und das Justierinstrument entfernt, der Zwischenwirbelraum gewünschtenfalls bearbeitet und die Zwischenwirbelprothese eingesetzt wird.

Die Erfindung wird im folgenden näher unter Bezugnahme auf die Zeichnung erläutert. Darin zeigen
- Fig. 1: das in einem Wirbelzwischenraum eingesetzte Justierinstrument,
- Fig. 2: eine entsprechende Darstellung mit einer auf die Justierstange aufgeschobenen Führungsein- richtung,
- Fig. 3: und 4 eine Schnitt- und Stirnansicht der Füh- rungseinrichtung,
- Fig. 5: eine Teil-Seitenansicht des Justierinstru- ments,
- Fig. 6: einen mit der Führungseinrichtung zu benutzen- den Bohrer,
- Fig. 7: einen mit der Führungseinrichtung zu benutzen- den Schraubendreher mit Schraubstift,
- Fig. 8: eine der Fig. 1 entsprechende Darstellung mit in die Wirbel eingesetzten Schraubstiften und
- Fig. 9: eine der Fig. 8 entsprechende Darstellung mit einem angesetzten Spreizinstrument und
- Fig. 10 - 18: die Veranschaulichung von Raspelwerkzeu- gen für eine bestimmte Prothese, nämlich:

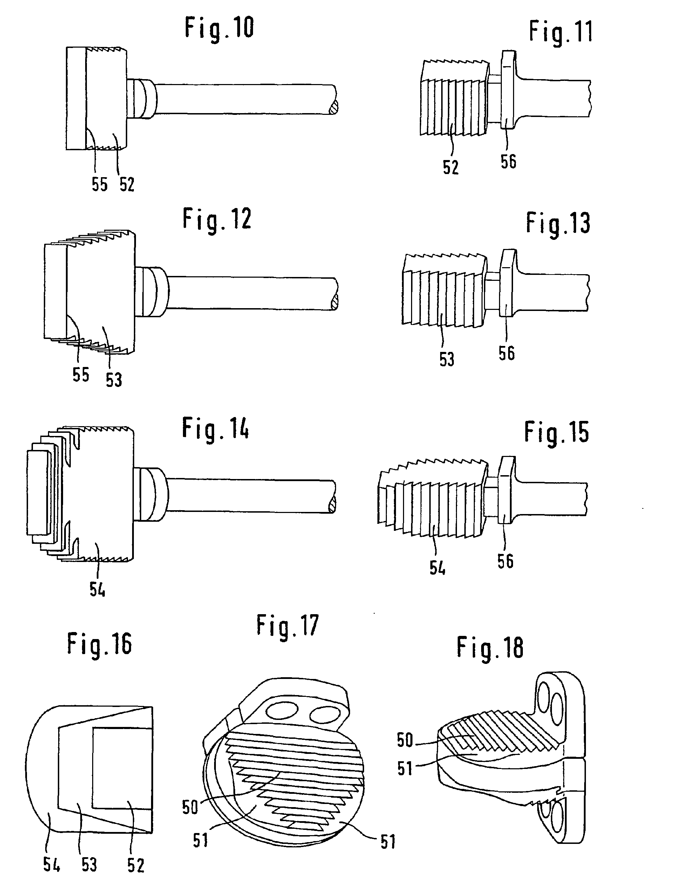

- Fig. 10 - 15: einen Satz von drei unterschiedliche Ras- peln,
- Fig. 16: den Umriß der Raspeln im Vergleich und
- Fig. 17 und 18: die Prothese, für die die Raspeln be- stimmt sind.

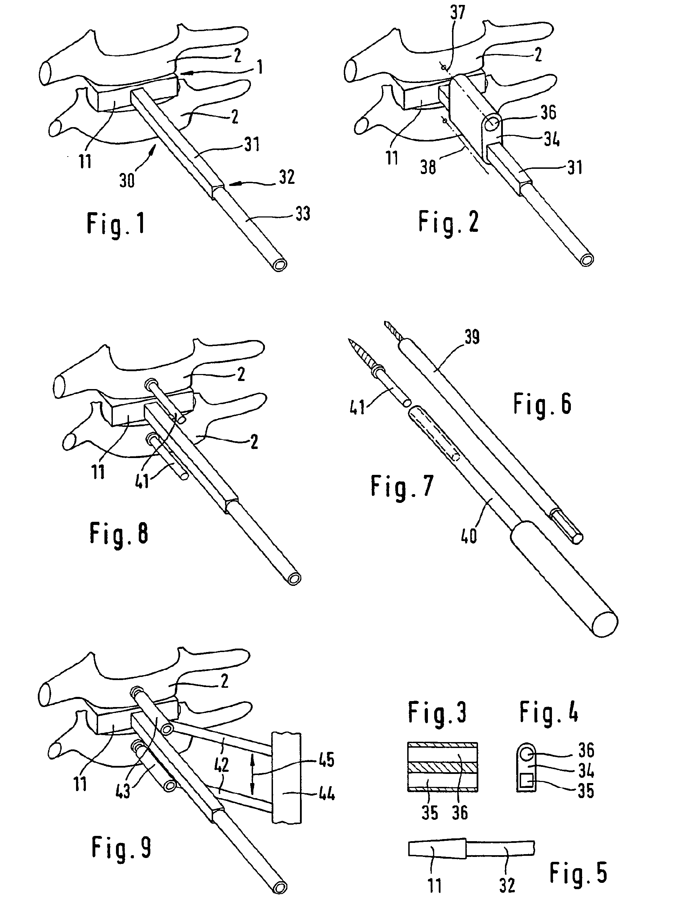

Das in Fig. 1-9 dargestellte Instrumentarium umfaßt ein Justierinstrument 30 mit einer Zwischenwirbelplatte 11. Sie hat eine Flächenausdehnung, die nur wenig geringer ist als die Flächenausdehnung des Zwischenwirbelraums, so daß sie darin zwischen den hochstehenden lateralen Randzacken selbsttätig positioniert wird. Sie hat etwa die Größe der später einzusetzenden Prothese. Ihre Abmessungen in AP- und LM-Richtung (LM = lateral-medial, d.h. lotrecht zur Medianebene) sind nicht wesentlich geringer als diejenigen des Zwischenwirbelraums. Ihre Abmessung in LM-Richtung soll mindestens 70 % des lichten Abstands zwischen den lateralen Randzacken des unteren Wirbelkörpers, vorzugsweise mindestens 80 %, betragen. Beim Eintreiben nimmt die Zwischenwirbelplatte daher eine etwa mittige Stellung ein. Mit einem Röntgengerät, das mindestens einen AP-Strahlengang aufweist, kann ihre mittige Lage und ihre Ausrichtung auf die Medianebene kontrolliert werden, wobei die Lage und Richtung der Außenflächen der Justierstange 32 maßgebend sind. Sie bilden die Röntgenmarkierung des Instruments für diese Kontrolle. Die Justierstange ist für diesen Zweck röntgen-opak ausgebildet, beispielsweise aus Metall. Ebenso kann in einem seitlichen Strahlengang kontrolliert werden, ob die Zwischenwirbelplatte die korrekte Tiefeneinstellung in AP-Richtung hat. Als Röntgenmarkierung dienen in diesem Fall die in LM-Richtung verlaufenden Kanten der Zwischenwirbelplatte oder eine besondere Markierung.

Die Dicke der Zwischenwirbelplatte 11 entspricht etwa der der einzusetzenden Zwischenwirbelprothese. Wenn unterschiedlich große Prothesen zur Auswahl stehen, werden auch entsprechend unterschiedliche Zwischenwirbelplatten im Instrumentarium angeboten. Sie ist jedenfalls so groß, daß sie nach der Entfernung der Bandscheibe in den Zwischenwirbelraum eingeschoben werden kann und dort durch die natürliche Bandspannung festgehalten wird. Sie kann in der Seitenansicht keilförmig ausgebildet sein, wie dies in Fig. 5 dargestellt ist. Wenn sie ihre vorgesehene Lage erreicht hat, hat sie eine genau definierte Lage zu den sie einschließenden Wirbelkörperoberflächen.

Dadurch ist Gewähr dafür gegeben, daß auch die von der Zwischenwirbelplatte 11 nach vorne ragende Justierstange 32 eine genau definierte Lage im Verhältnis zu den Wirbelkörpern aufweist. Diese hat zumindest in ihrem der Zwischenwirbelplatte 11 nahen Bereich 31 einen quadratischen Querschnitt, während der weiter entfernte Abschnitt 33 mit einem Rundquerschnitt dargestellt ist. Die Justierstange 32 ist als Rohr ausgeführt, um als Röntgenmarkierung für einen in AP-Richtung verlaufenden Röntgenstrahlengang dienen zu können. Die Rohrform ist jedoch nicht erforderlich, weil auch die äußere Kontur der Justierstange als Röntgenmarkierung dienen kann.

Die Justierstange 32 dient zur Justierung (Positionierung) der Führungseinrichtung 34. In Fig. 3 und 4 erkennt man, daß diese eine Bohrung 35 mit quadratischem Querschnitt aufweist, die passend zu dem Abschnitt 31 der Justierstange 32 ausgeführt ist. Dieser Teil der Führungseinrichtung bildet eine Nabe, durch die sie auf der Justierstange gehalten wird. Sie enthält ferner eine zu der Bohrung 35 parallele Bohrung 36 mit rundem Querschnitt. Diese Bohrung 36 dient als Bohrlehre oder allgemeiner zur Führung von Bearbeitungswerkzeugen. Wenn die Führungseinrichtung 34 auf den Abschnitt 31 der Justierstange aufgeschoben ist, kann sie die in Fig. 2 dargestellte Stellung einnehmen, in welcher die von der Bohrung 36 definierte Führungsachse 37 auf die Mitte des oberen Wirbelkörpers 2 zielt. Sie kann außerdem eine um 180° dazu gedrehte Stellung einnehmen, in welcher die von der Bohrung 36 definierte Führungsachse 38 auf die Mitte des unteren Wirbelkörpers zielt. Die Achsen 37 und 38 liegen in derselben Medianebene wie die Justierstange 32. Statt des quadratischen Querschnitts im Abschnitt 31 kann auch eine andere unrunde Form gewählt werden, die die Möglichkeit des Zusammenwirkens in zwei um 180° zueinander versetzten Positionen der Führungseinrichtung 34 erlaubt.

Die Führungseinrichtung 34 wird in der Weise benutzt, daß sie zunächst zur Bearbeitung des einen Wirbels verwendet wird und dann, nachdem sie auf den Abschnitt 33 mit rundem Querschnitt der Justierstange 32 zurückgezogen wurde, um 180° zur Bearbeitung des anderen Wirbels gewendet wird.

Die Bearbeitung besteht darin, daß zunächst mittels eines Bohrers 39, dessen Schaft passend zur Bohrung 36 ausgebildet ist, in einem Wirbel eine Bohrung eingebracht wird, in die anschließend mittels des Schraubendrehers 40, dessen Schaft gleichfalls zur Bohrung 36 paßt, ein Schraubstift 41 eingebracht wird. Der Stift 41 paßt genau in eine im Schraubendreher 40 vorgesehene Bohrung, so daß Gewähr dafür vorhanden ist, daß er fluchtend mit dem Schraubendreher 40 und somit auch fluchtend mit den Achsen 37, 38 und parallel zur Justierstange 32 in den jeweiligen Wirbel eingeschraubt wird. Nachdem dies an beiden Wirbeln geschehen ist, bietet sich das in Fig. 8 dargestellte Bild. Die Schraubstifte 41 ragen dank der Führungseinrichtung und deren Justierung durch das Justierinstrument genau in der Medianebene und parallel zueinander von den beiden Wirbeln 2 in ventrale Richtung.

Es kann nun ein Distraktionsinstrument an die Stifte 41 angesetzt werden, das an zwei Armen 42 je eine Aufnahme 43 für die Stifte 41 aufweist, wobei die Arme 42 an einem Instrumentenkörper 44 parallel zueinander in Pfeilrichtung 45 distrahiert und positioniert werden können. Derartige Distraktionsinstrumente sind bekannt und bedürfen daher hier keiner Beschreibung.

Die Wirbel 2 können mit Hilfe dieses Instruments - falls erforderlich - noch ein wenig distrahiert werden, damit die Zwischenwirbelplatte 11 entnommen werden kann. Gewünschtenfalls kann der Zwischenwirbelraum in dem Zustand, in welchem die Wirbel durch das Instrument 42 bis 44 und die Stifte 41 gehalten sind, zur Vorbereitung der Aufnahme der Zwischenwirbelprothese weiter bearbeitet werden. Schließlich wird die Prothese in den Zwischenwirbelraum eingesetzt und erhält ihre endgültige Position dadurch, daß die Distraktion der Wirbel 2 mit dem Instrument 42 bis 44 rückgängig gemacht wird.

Das Instrumentarium umfaßt auch einen Satz von Raspeln, die die Oberflächenform der Wirbel zur Aufnahme der Prothese vorbereiten. Sie sind in Fig. 10 bis 16 dargestellt. Die gezeigten Beispiele sind auf das Ausführungsbeispiel der Prothese abgestimmt, die in Fig. 17 und 18 dargestellt ist. Sie hat einen ovalen bis rechteckigen Umriß, der zur weitgehenden Ausnutzung der Ausdehnung des Zwischenwirbelraums bestimmt ist. Sie ist so flach, daß sie ohne tiefgreifende Abfräsung der Wirbelkörperdeckplatten eingesetzt werden kann. Sie wendet den Wirbelkörperdeckplatten Außenflächen zu, die in ihrem größten Teil 50 etwa eben und gezahnt sind. Ihre dorsolateralen Ecken 51 sind derart abgeschrägt, daß die Oberfläche in diesen Bereichen gegenüber der Ebene des Flächenanteils 50 zurücktritt.

Eine komplementäre Form des Zwischenwirbelraums wird durch einen Satz von Raspeln 52, 53 und 54 vorbereitet, die in den Figuren 10 bis 15 dargestellt sind. Die abgestuften Größenverhältnisse der Raspeln zeigt Fig. 16. Zuerst wird die kleinste Raspel 52 mittels eines nicht detailliert dargestellten Griffs in den Zwischenwirbelraum eingestoßen, um den Zugang zu eröffnen. Es folgt Raspel 53, die Trapezform aufweist, etwa entsprechend der Trapezform des ebenen Flächenanteils der Prothesenoberfläche. Schließlich formt Raspel 54 den Zwischenwirbelraum im wesentlichen übereinstimmend mit der Form der einzusetzenden Prothese. Die Höhe aller Raspeln gleicht derjenigen der Prothese. Alle Raspeln sind in denjenigen Flächen, die dem ebenen Anteil 50 der Prothese entsprechen, ungezahnt ausgebildet. Das bedeutet, daß sie hauptsächlich mit Ihrer Vorderkante 55 eine Abrasion des Knorpels und eine Anfrischung des Knochens durchführen, ohne die Wirbelkörperendfläche wesentlich abzutragen. Alle Raspeln sind mit einem Anschlag 56 versehen, der dafür sorgt, daß sie nur bis zur vorgesehenen Tiefe in den Zwischenwirbelraum eindringen können.

## Patentansprüche

1. Instrumentarium zum Einsetzen einer Zwischenwirbelprothese in einen Zwischenwirbelraum (1) zwischen zwei Wirbelkörpern (2), das
a)eine Justiereinrichtung (30), die aus einer Zwischenwirbelplatte (11) und einer davon vorragenden Justierstange (32) besteht,
b)eine von der Justierstange (32) lösbar getragene Führungseinrichtung (34), die zwei in der Medianebene unterhalb und oberhalb der Justierstange (32) liegende Führungsachsen (37,38) für ein Bearbeitungsinstrument (39, 40) bildet, und
c)zwei mittels des Bearbeitungsinstruments (39,40) in die Wirbelkörper (2) in Richtung der Führungsachsen (37, 38)einbringbare Haltemittel (41) aufweist,
**dadurch gekennzeichnet, daß**
ein die Haltemittel (41) richtungsfest haltendes Distraktionsinstrument (42, 43, 44) vorgesehen ist.

2. Instrumentarium nach Anspruch 1, **dadurch gekennzeichnet, daß** eine Nabe der Führungseinrichtung (34) von dem freien Ende der Justierstange (32) her auf diese aufschiebbar ist und die Justierstange (32) und die Nabe undrehbar komplementär passend zueinander geformte Zusammenwirkungsflächen (31, 35) aufweisen.

3. Instrumentarium nach Anspruch 2, **dadurch gekennzeichnet, daß** die Führungseinrichtung (34) je eine Führung oberhalb und unterhalb der Justierstange aufweist.

4. Instrumentarium nach Anspruch 2, **dadurch gekennzeichnet, daß** die Führungseinrichtung (34) nur eine Führung (36) aufweist und die komplementär zusammenwirkenden Flächen (31, 35) der Justierstange (32) und der Nabe in um 180° zueinander versetzten Stellungen zusammensteckbar sind.

5. Instrumentarium nach Anspruch 2, **dadurch gekennzeichnet, daß** die die Führungsachsen (37, 38) parallel zu der Justierstange (32) verlaufen.

6. Instrumentarium nach Anspruch 5, **dadurch gekennzeichnet, daß** die Haltemittel Stifte (41) sind.

7. Instrumentarium nach Anspruch 1, **dadurch gekennzeichnet, daß** die Flächenausdehnung der Zwischenwirbelplatte (11) wenig geringer ist als die Flächenausdehnung des Zwischenwirbelraums.

8. Instrumentarium nach Anspruch 1, **dadurch gekennzeichnet, daß** die Oberfläche der Zwischenwirbelplatte (11) so ausgebildet ist, daß sie unter Röntgenkontrolle im Zwischenwirbelraum (1) in Querrichtung verschiebbar und positionierbar ist.

9. Instrumentarium nach Anspruch 1, **dadurch gekennzeichnet, daß** die Zwischenwirbelplatte (11) keilförmig ist.

## Claims

1. Instrument set for inserting an intervertebral prosthesis into an intervertebral space (1) between two vertebral bodies (2), which instrument set has
a) an adjustment device (30) consisting of an intervertebral plate (11) and of an adjustment rod (32) projecting from the latter,
b) a guide device (34) which is supported in a detachable manner by the adjustment rod (32) and which forms two guide axes (37, 38) for a working instrument (39, 40), said guide axes (37, 38) lying in the median plane below and above the adjustment rod (32), and
c) two holding means (41) which can be introduced in the direction of the guide axes (37, 38) into the vertebral bodies (2) by means of the working instrument (39, 40),
**characterized in that** a distraction instrument (42, 43, 44) which maintains the holding means (41) in a fixed direction is provided.

2. Instrument set according to Claim 1, **characterized in that** a hub of the guide device (34) can be pushed onto the adjustment rod (32) from the free end thereof, and the adjustment rod (32) and the hub have interacting surfaces (31, 35) shaped so as to complement one another and give a non-rotational fit.

3. Instrument set according to Claim 2, **characterized in that** the guide device (34) has one guide above the adjustment rod and one guide below the adjustment rod.

4. Instrument set according to Claim 2, **characterized in that** the guide device (34) has only one guide (36), and the complementary interacting surfaces (31, 35) of the adjustment rod (32) and of the hub can be fitted together in positions offset 180° in relation to one another.

5. Instrument set according to Claim 2, **characterized in that** the guide axes (37, 38) run parallel to the adjustment rod (32).

6. Instrument set according to Claim 5, **characterized in that** the holding means are pins (41).

7. Instrument set according to Claim 1, **characterized in that** the surface area of the intervertebral plate (11) is slightly smaller than the surface area of the intervertebral space.

8. Instrument set according to Claim 1, **characterized in that** the surface of the intervertebral plate (11) is designed such that it is displaceable in the transverse direction and positionable in the intervertebral space (1) under X-ray control.

9. Instrument set according to Claim 1, **characterized in that** the intervertebral plate (11) is wedgeshaped.

## Revendications

1. Instrument d'insertion d'une prothèse intervertébrale dans une région intervertébrale (1) entre deux corps de vertèbre (2), qui comprend
a) un dispositif d'ajustage (30), qui se compose d'une plaque intervertébrale (11) et d'une tige d'ajustage (32) saillante sur celle-ci;
b) un dispositif de guidage (34) porté de façon détachable par la tige d'ajustage (32), qui forme deux axes de guidage (37, 38) pour un instrument de façonnage (39, 40), situés en dessous et au-dessus de la tige d'ajustage (32) dans le plan médian, et
c) deux moyens de maintien (41) pouvant être introduits dans les corps de vertèbre (2) dans
la direction des axes de guidage (37, 38) au moyen de l'instrument de façonnage (39, 40), **caractérisé en ce qu'**il est prévu un instrument de retenue (42, 43, 44) maintenant fermement la direction des éléments de maintien (41).

2. Instrument selon la revendication 1, **caractérisé en ce qu'**un moyeu du dispositif de guidage (34) peut être déplacé sur la tige d'ajustage (32) à partir de l'extrémité libre de celle-ci et la tige d'ajustage (32) et le moyeu présentent des faces de collaboration (31, 35) sans rotation dont les formes s'adaptent l'une à l'autre de manière complémentaire.

3. Instrument selon la revendication 2, **caractérisé en ce que** le dispositif de guidage (34) présente chaque fois un guidage au-dessus et en dessous de la tige d'ajustage.

4. Instrument selon la revendication 2, **caractérisé en ce que** le dispositif de guidage (34) ne présente qu'un seul guidage (36) et les faces de collaboration complémentaires (31, 35) de la tige d'ajustage (32) et du moyeu peuvent être engagées dans des positions tournées de 180° l'une par rapport à l'autre.

5. Instrument selon la revendication 2, **caractérisé en ce que** les axes de guidage (37, 38) s'étendent parallèlement à la tige d'ajustage (32).

6. Instrument selon la revendication 5, **caractérisé en ce que** les moyens de maintien sont des broches (41).

7. Instrument selon la revendication 1, **caractérisé en ce que** l'extension de surface de la plaque intervertébrale (11) est un peu plus petite que l'extension de surface de la région intervertébrale.

8. Instrument selon la revendication 1, **caractérisé en ce que** la surface de la plaque intervertébrale (11) est réalisée de telle manière qu'elle puisse être déplacée et positionnée en direction transversale dans la région intervertébrale (1) sous un contrôle aux rayons X.

9. Instrument selon la revendication 1, **caractérisé en ce que** la plaque intervertébrale (11) est réalisée en forme de coin.
